# NEUE EUROPÄISCHE PATENTSCHRIFT

(11) **EP 1 796 794 B2**
(45) Veröffentlichungstag und Bekanntmachung des Hinweises auf die Entscheidung über den Einspruch: **10.11.2010**
(45) Hinweis auf die Patenterteilung: 09.01.2008
(21) Anmeldenummer: 06776412.6
(22) Anmeldetag: 26.07.2006
(51) Int. Cl.: A61Q 3/02, A61K 8/26, A61K 8/55

(54) **KOSMETISCHE ZUSAMMENSETZUNG ZUR ERZEUGUNG EINES KOSMETISCHEN ÜBERZUGS MIT EINEM METALLISCH GLÄNZENDEN ERSCHEINUNGSBILD, KÜNSTLICHER FINGERNAGEL SOWIE VERWENDUNG VON PVD-ALUMINIUMPIGMENTEN**
COSMETIC COMPOSITION FOR OBTAINING A COSMETIC COATING WITH A METALLICALLY GLOSSY APPEARANCE, ARTIFICIAL FINGERNAILS AND USE OF PVD ALUMINIUM PIGMENTS
COMPOSITION COSMETIQUE POUR PRODUIRE UN REVETEMENT COSMETIQUE D'ASPECT METALLIQUE BRILLANT, ONGLE ARTIFICIEL, AINSI QU'UTILISATION DE PIGMENTS D'ALUMINIUM PVD

(30) Priorität: 29.07.2005 DE 102005036333
(43) Veröffentlichungstag der Anmeldung: 20.06.2007
(73) Patentinhaber: Eckart GmbH, 91235 Hartenstein (DE)
(72) Erfinder: KRÜGER, Peter, 91207 Lauf (DE); VOIT, Thomas, 91275 Auerbach (DE)
(74) Vertreter: Walcher, Armin
(86) Internationale Anmeldenummer: PCT/EP2006/007354
(87) Internationale Veröffentlichungsnummer: WO 2007/014680

(56) Entgegenhaltungen:
- EP-A- 0 826 745
- WO-A-02/03913
- WO-A-2005/055965
- DE-A1- 10 315 775

## Beschreibung

Die Erfindung betrifft eine kosmetische Zusammensetzung zur Erzeugung eines kosmetischen Überzugs mit einem metallisch glänzenden Erscheinungsbild.

Aus der WO 02/03913 ist ein Nagellack, der einem Fingernagel ein metallisches Erscheinungsbild verleiht, bekannt. Bei dieser bekannten Nagellack-Formulierung werden PVD-Pigmente verwendet. PVD-Pigmente besitzen zwar aufgrund ihrer extrem glatten Oberfläche einen starken Spiegelglanz, weisen jedoch in Applikationen einen non-leafing-Effekt auf. Dies bedeutet, dass sie im flüssigen Lackfilm absinken und dadurch der brillante Effekt vermindert wird.

Auch die US 2004/0241423 beschreibt einen Nagellack mit einem Spiegeleffekt. Ausweislich Absatz [0004] der Beschreibung wird ein gegenüber der WO02/03913 verbesserter Spiegeleffekt dadurch erreicht, dass die Zubereitung insbesondere eine niedrige Viskosität und einen hohen Gehalt an metallischen Teilchen aufweist.

Jedoch ist es angesichts der hohen Preise für PVD-Pigmente wünschenswert, einen befriedigenden Effekt bei einem geringen Gehalt an Metallpigmenten zu erzielen. Zudem wird durch die in der US 2004/0241423 beschriebene Vorgehensweise zwar eine verbesserte Orientierung, jedoch kein leafing-Effekt erzielt.

Es besteht mithin ein Bedarf an kosmetischen Formulierungen, die nach Aufbringung einen Überzug mit einem extremen Spiegelglanz erzeugen.
Aufgabe der vorliegenden Erfindung ist es, eine kosmetische Zusammensetzung bereitzustellen, die nach der Applikation einen stärkeren Spiegelglanz erzeugt, als es mit bislang bekannten kosmetischen Zusammensetzungen möglich ist, der den Eindruck einer vorzugsweise geschlossenen Metallschicht bietet.

Die der Erfindung zugrunde liegende Aufgabe wird gelöst durch Bereitstellung einer kosmetischen Zusammensetzung, umfassend eine Flüssigphase und PVD-Aluminiumpigment, wobei die kosmetische Zusammensetzung das PVD-Aluminiumpigment in einer Pigmentierungshöhe von 0,05 bis 5,0 Gew.-%, bezogen auf das Gesamtgewicht der kosmetischen Zusammensetzung, und wenigstens ein leafing-Additiv umfasst.

Bevorzugte Weiterbildungen sind in den Unteransprüchen angegeben.

Nach Aufbringung der erfindungsgemäßen kosmetischen Zusammensetzung kann ein kosmetischer Überzug mit einem metallisch glänzenden Erscheinungsbild erzeugt werden.

Ausgehend von bekannten PVD-Aluminiumpigmenten (beispielsweise Metalure®, vertrieben durch die Firma ECKART GmbH & Co. KG), die trotz ihres non-leafing-Verhaltens bereits einen Spiegelglanz aufweisen, wird den Pigmenten durch Zusatz eines oder mehrerer leafing-Additive ein leafing-Verhalten verliehen, was dazu führt, dass die Pigmente an der Oberfläche des Lackfilms aufschwimmen und einen stark verbesserten, extremen Spiegelglanz aufweisen. Das leafing-Additiv bindet an die Oberfläche des PVD-Aluminiumpigmentes, worauf die PVD-Aluminiumpigmente sich an der Oberfläche oder in der Nähe der Oberfläche einer Flüssigkeit anordnen.

Die Aufgabe wird ferner durch Bereitstellung eines künstlichen Fingernagels gemäß Anspruch 21 gelöst. Im Sinne der Erfindung wird unter dem Begriff "künstlicher Fingernagel" auch ein künstlicher Fußnagel verstanden.

Die der Erfindung zugrundeliegende Aufgabe wird auch durch die Verwendung von PVD-Aluminiumpigment mit leafing-Eigenschaften in einer kosmetischen Zusammensetzung, vorzugsweise einem Nagellack, gelöst, wobei das PVD-Aluminiumpigment oberflächlich mit einem leafing-Additiv versehen ist.

Unter PVD-Aluminiumpigment wird im Sinne der Erfindung verstanden, dass das Aluminiumpigment unter Verwendung eines PVD-Verfahrens (PVD: physical vapour deposition) hergestellt worden ist. Mittels PVD-Verfahren lassen sich sehr dünne Aluminiumpigmente mit einer äußerst glatten und spiegelnden Oberfläche herstellen. PVD-Verfahren zur Herstellung von Aluminiumpigmenten sind dem Fachmann beispielsweise aus der EP 0 826 745 bekannt, die hiermit unter Bezugnahme aufgenommen ist.

Die bei der vorliegenden Erfindung verwendeten PVD-Aluminiumpigmente weisen keine Beugungsstrukturen oder Beugungselemente auf. Die Oberfläche der vorliegende verwendeten PVD-Aluminiumpigmente sind glatt und eben und erzeugen daher den gewünschten metallischen Spiegelglanz.

Diese PVD-Aluminiumpigmente werden bevorzugt in stark verdünnten Dispersionen mit organischen Lösemitteln gehandhabt, um starke Agglomeration zu vermeiden.

Es hat sich nunmehr völlig überraschend gezeigt, dass man durch Zusatz von leafing-Additiven diesen PVD-Aluminiumpigmenten, die stets non-leafing-Eigenschaften aufweisen, leafing-Eigenschaften verleihen kann.

Bei diesen Pigmenten handelt es sich um ein handelsübliches PVD-Aluminiumpigment, beispielsweise Metalure®, vertrieben durch ECKART GmbH & Co. KG, und es war für den Fachmann nicht zu erwarten, dass sich durch einfaches Zugeben eines leafing-fördernden Stoffes oder eines leafing-Additivs ein leafing-Effekt in einer kosmetischen Zusammensetzung einstellt.
Bei solchen leafing-fördernden Stoffen ("leafing-Additiven") handelt es sich bevorzugt um langkettige Phosphorsäureester oder Gemischen von Phosphorsäureestern. Erfindungsgemäß haben sich Phosphorsäureester mit einer Kohlenstoffkettenlänge von 12 bis 24 Kohlenstoffatomen als sehr geeignet erwiesen. Besonders bevorzugt sind -Phosphorsäureester oder Gemische von Phosphorsäreestern, bei denen die Kohlenstoffkette eine Länge von 14 bis 18 Kohlenstoffatomen aufweist. Die Kohlenstoffkette kann linear oder verzweigt sein. Vorzugsweise ist die Kohlenstoffkette linear.

Bei den Phosphorsäureestern kann es sich um Mono-, Di- und/oder Triester handeln, wobei Phosphorsäuremonoester besonders bevorzugt sind.

Bevorzugt sind die Phosphorsäureester Fettsäurephosphorsäureester, bevorzugt Fettsäurephosphorsäuremonoester.

Als sehr geeignet haben sich Myristin-, Pentadecan-, Palmitin-, Margarin- und Stearinsäurephosphorsäureester erwiesen, die einzeln oder als Gemisch als leafing-Additiv verwendet werden können.

Es hat sich völlig überraschend gezeigt, dass die gewünschte Wirkung (leafing-Effekt) bereits durch einfache Zugabe des leafing-Additivs zur Dispersion des PVD-Pigments erzielt werden kann. Es ist mithin überraschenderweise nicht erforderlich, die PVD-Aluminiumpigmente vor der Zugabe zur der kosmetischen Zusammensetzung mit dem leafing-Additiv zu beschichten.

Die Erfindung betrifft mithin eine kosmetische Zusammensetzung, welche nach Aufbringung einen Überzug mit vorzugsweise geschlossen erscheinendem Metallfilm bereitstellt, der einen bisher nie erreichten Spiegelglanz aufweist.

Unter einer kosmetischen Zusammensetzung werden im Sinne der Erfindung sämtliche kosmetischen Formulierungen verstanden, die eine Flüssigphase aufweisen, in der die PVD-Aluminiumpigmente leafing-Eigenschaften zeigen und die auf im Wesentlichen flächige Untergründe aufgetragen werden. Vorzugsweise werden die kosmetischen Zubereitungen aus der Gruppe ausgewählt, die aus flüssigem Make-up, flüssigem Eyeliner, flüssigem Lidschatten, Körperlotion, Parfüm, Lipgloss und Nagellack besteht.

Vorzugsweise ist die kosmetische Zusammensetzung ein Nagellack.

Bei der erfindungsgemäßen kosmetischen Zusammensetzung tritt das metallisch glänzende Erscheinungsbild nach Auftragen der kosmetischen Zusammensetzung auf einen flächigen Untergrund auf. In der kosmetischen Zusammensetzung selbst ist der Effekt nicht zwangsläufig in gleicher Weise sichtbar. Nach dem Auftragen der erfindungsgemäßen kosmetischen Zusammensetzung kommt es zu einem Aufschwimmen der PVD-Aluminiumpigmente auf der Oberfläche des vorzugsweise flüssigen Lackfilms, beispielsweise auf natürlichen oder künstlichen Fingernägeln, auf Lippen, Augenlidern, Wangen oder anderen flächigen Körperbereichen. Um diese Orientierung der Aluminiumpigmente zu ermöglichen, ist es erforderlich, dass die kosmetische Zusammensetzung eine Flüssigphase aufweist.

Kosmetische Zusammensetzungen, die keine Flüssigphase enthalten, eignen sich vorwiegend nicht, da eine Flüssigphase erforderlich ist, um ein Aufschwimmen der Aluminiumpigmente und damit den gewünschten metallisch glänzenden Effekt in dem aufgebrachten Überzug zu ermöglichen.

Das heißt, für eine Orientierung der Aluminiumpigmente ist ein ausreichender Feuchtigkeits- bzw. Lösemittelgehalt in der kosmetischen Zusammensetzung nötig, um nach dem Auftragen der Zusammensetzung und Verdunsten des Lösemittels eine Orientierung der Effektpigmente zu ermöglichen. Ferner kann die Orientierung der PVD-Aluminiumpigmente verbessert bzw. unterstützt werden, wenn während des Auftragens der kosmetischen Zusammensetzung eine mechanische Einwirkung erfolgt. Dies ist beispielsweise beim Auftragen eines Nagellacks mit einem Pinsel auf natürliche oder künstliche Fingernägel gegeben. Beim Aufbringen eines Lipgloss auf die Lippen wird die Orientierung beispielsweise durch die Auftrags- bzw. Rollkugel des Lipgloss-Auftragsstiftes unterstützt. Alternativ kann der Lipgloss auch mittels Pinselauftrag auf die Lippen aufgebracht werden.

Als Flüssigphase werden vorzugsweise flüssige Komponenten verwendet, die für die jeweilige Applikation gesundheitlich unbedenklich sind. Das heißt, bei einem Nagellack können organische Lösemittel verwendet werden, die bei einem Lipgloss gegebenenfalls nur eingeschränkt oder gar nicht verwendet werden können. Der Fachmann kann jedoch ohne weiteres eine für den jeweiligen Verwendungszweck geeignete Flüssigphase, beispielsweise ein organisches Lösemittel, Wasser, Öl, Gel oder geeignete Mischungen davon auswählen.

Eine erfindungsgemäße Nagellackzusammensetzung besteht in der Regel aus für diesen Verwendungszweck nicht-toxischen Komponenten und ist geeignet, einen Filmüberzug mit metallisch glänzendem Effekt auf natürlichen oder künstlichen Fingernägeln auszubilden. Der Begriff "nicht-toxisch" bezieht sich hierbei im allgemeinen auf die in der EG-Richtlinie 761768/EG aufgeführten Stoffe.

Eine Nagellackzusammensetzung enthält vorzugsweise ein Lösemittel, wenigstens eine filmbildende Komponente sowie ein PVD-Aluminiumpigment sowie ein leafing-Additiv.

Die erfindungsgemäße kosmetische Zusammensetzung zur Erzeugung eines kosmetischen Überzugs mit einem metallisch hochglänzenden Erscheinungsbild umfasst eine Flüssigphase und PVD-Aluminiumpigment, wobei das PVD-Aluminiumpigment leafing-Eigenschaften aufweist und in der kosmetischen Zusammensetzung in einer Pigmentierungshöhe von 0,05 bis 5,0 Gew.-%, bezogen auf das Gesamtgewicht der kosmetischen Zusammensetzung, enthalten ist, sowie ein leafing-förderndes Additiv.

Die Pigmentierungshöhe des PVD-Aluminiumpigments beträgt 1 bis 2 %, bevorzugt 1,3 bis 1,8 Gew.-%, bezogen auf das Gesamtgewicht der kosmetischen Zusammensetzung.

Der Festkörpergehalt der kosmetischen Zusammensetzung liegt vorzugsweise zwischen 5 und 17 Gew.-%, bezogen auf das Gesamtgewicht der kosmetischen Zusammensetzung.

Bei der erfindungsgemäßen kosmetischen Zusammensetzung handelt es sich bevorzugt um einen Nagellack. Dieser kann eine filmbildende Komponente enthalten, die beispielsweise aus der Gruppe enthaltend Nitrocellulose, Polyesterharze, Polyvinylharze, Alkydharze, Epoxidharze oder Celluloseacetatbutyrat und geeignete, nicht-toxische Derivate sowie ihre Mischungen ausgewählt wird. Bevorzugt sind Celluloseacetatbutyrat sowie Nitrocellulose. Vorzugsweise hat die Nitrocellulose ein Molekulargewicht von wenigstens 56.000 g/mol, weiter bevorzugt von wenigstens 112.000 g/mol. Celluloseacetatbutyrat weist vorzugsweise ein Molekulargewicht von mehr als 83.000 g/mol auf.

Das PVD-Aluminiumpigment wird vorzugsweise in Form einer Dispersion in einem organischen Lösemittel bei der Herstellung einer kosmetischen Zusammensetzung verwendet, der ein leafing-Additiv zugesetzt wird.

Dies sind bevorzugt langkettige (C12 - C24) Phosphorsäureester, besonders bevorzugt sind C14-C18-Phosphorsäureester. Selbst durch einfache Zugabe des leafing-Additivs zur Dispersion des PVD-Aluminiumpigments kann die gewünschte Wirkung erzielt werden. Bevorzugt lässt man diese Dispersion dann vor der Weiterverarbeitung für einige Zeit stehen.

Der Feststoffgehalt der einer kosmetischen Zusammensetzung zuzusetzenden Dispersion kann 1 bis 15 Gew.-%, bevorzugt 3 bis 10 Gew.-%, bezogen auf das Gesamtgewicht der kosmetischen Zusammensetzung,
betragen. Bei höheren Konzentrationen von PVD-Aluminiumpigmenten besteht die Gefahr einer Agglomeration der Aluminiumpigmente.

Bei der Wahl der Lösemittel hat sich überraschend gezeigt, dass insbesondere Ketone und Acetate mit einem Molgewicht unter 120 g/mol für die Erzielung eines leafing-Effekts geeignet sind. Beispielsweise können Methylisobutylketon, Methylethylketon, Ethylacetat, Butylacetat, Methylacetat, Aceton oder Gemische daraus verwendet werden. Bevorzugt für Nagellackzusammensetzungen sind Methylethylketon oder Ethylacetat.
Im Einzelfall kann es erforderlich sein, das Lösemittel auf das verwendete leafing-Additiv abzustimmen.

Hiermit erfindungsgemäß hergestellte kosmetische Zusammensetzungen, insbesondere Nagellackzusammensetzungen, weisen einen zuvor nicht gekannten Glanz auf.

Als organische Lösemittel in der Nagellackzusammensetzung können aber auch weitere Lösemittel wie beispielsweise Toluol, Ethanol, Hexan, Heptan, Cyclopentan, Cyclohexan, zyklische Ether wie z.B. Tetrahydrofuran oder 1,4-Dioxan, Cellosolveacetat, Ethylcellosolve, Butylcellosolve und Gemische hiervon zur Anwendung kommen.

Der Lösemittelgehalt der erfindungsgemäßen Nagellackzusammensetzungen beträgt vorzugsweise 50 bis 90 Gew.-%, weiter bevorzugt 60 bis 85 Gew.-% und noch weiter bevorzugt 65 bis 75 Gew.-%, jeweils bezogen auf das Gesamtgewicht der Nagellackzusammensetzung.

So ist es möglich, Nagellackzusammensetzungen zu erhalten, die in der Applikation einen bisher nicht gekannten Spiegelglanz zeigen.

Gemäß einer bevorzugten Weiterbildung der Erfindung kann die Nagellackzusammensetzung einen Weichmacher und/oder ein Dispergiermittel enthalten.

Weichmacher machen insbesondere das Bindemittel weicher und besser verarbeitbar. Es kann ein Weichmacher oder aber auch eine Kombination mehrerer Weichmacher verwendet werden. Beispiele für derartige Weichmacher sind:
Campher, Rizinusöl, Ester der Zitronen-, Stearin-, Öl-, Phthal-, und Benzoesäure sowie ihre Derivate.

Als Phthalate werden vorzugsweise Dibutylphthalat, Diethylphthalat, Diamylphthalat, Dioctylphthalat und Dibutoxyethylphthalat oder Mischungen davon verwendet. Alternativ für die toxikologisch bedenklichen Phthalate können aber auch andere Weichmacher wie beispielsweise Glyceryltriacetat, Glyceryltripropionat, Glyceryltribenzoat, Dibutyltartrat, Benzylbenzoat, Tricresylphosphat, Tributylphosphat, Triphenylphosphat, Butylacetylricinoleat, Butylstearat, Triethylcitrat, Acetyltributylcitrat, Saccharoseacetatisobutyrat, Butylglycolat, Glycerintriacetat und Glycerintriproprionat oder Gemische davon verwendet werden.

Gemäß einer weiteren Ausführungsform der Erfindung können Dispergieradditive zugesetzt werden, um die PVD-Aluminiumpigmente besser zu dispergieren. Bevorzugte Beispiele derartiger Dispergiermittel sind Montmorillonit-Tone wie z.B. Bentonite und insbesondere Stearylalkoniumhectorit und/oder Stearylalkoniumbentonit. Als Dispergiermittel kann auch polymerer Harnstoff, optional in Kombination mit Bentoniten, verwendet werden.

Als weitere Zusätze können andere Effektpigmente und/oder Farbmittel wie Farbpigmente und/oder Farbstoffe zu der kosmetischen Zusammensetzung zugegeben werden.

Weiterhin kann die erfindungsgemäße Nagellackzusammensetzung weitere Additive wie Thixotropiemittel, Antioxidantien, Emulgatoren, Vitamine, Duftstoffe, Lichtschutzmittel, Konservierungsstoffe, Füllstoffe und/oder Medikamente enthalten. Diese Additive sind im Stand der Technik bekannt und werden gegebenenfalls in den üblichen Mengen dazugegeben.

Bevorzugt umfasst eine erfindungsgemäße Nagellackzusammensetzung, die vorzugsweise aus nicht-toxischen Komponenten besteht, PVD- Aluminiumpigment, mit einem metallischen Aluminiumanteil von 90 bis 99,9 Gew.-%, bezogen auf das Gesamtgewicht des Aluminiumpigments, in einer Pigmentierungshöhe von 0,05 bis maximal 5 Gew.-%, bezogen auf das Gesamtgewicht des Nagellacks, und wenigstens ein leafing-Additiv. Weiter bevorzugt ist eine Pigmentierungshöhe von 0,1 bis 2 Gew.-%, bezogen auf das Gesamtgewicht des Nagellacks.

Es hat sich gezeigt, dass das PVD-Aluminiumpigment in der kosmetischen Zusammensetzung mit sehr hoher Ergiebigkeit, d. h. hervorragender Deckkraft, verwendet werden kann. Kosmetische Zusammensetzungen mit einem PVD-Aluminiumpigment weisen eine äußerst hohe Deckkraft auf. Daher ist das PVD-Aluminiumpigment vorteilhaft in sehr niedrigen Pigmentierungshöhen in der erfindungsgemäßen kosmetischen Zusammensetzung verwendbar.

Durch PVD-Verfahren hergestellte Aluminiumpigmente sind auf Grund ihrer geringen Dicke so flexibel, dass sie sich perfekt an einen Untergrund anpassen, d.h. sich quasi an den Untergrund anschmiegen.

Es hat sich bei der vorliegenden Erfindung gezeigt, dass die Agglomerationsneigung in der kosmetischen Zusammensetzung, vorzugsweise einem Nagellack oder einem Lipgloss, bei gleichzeitiger Verwendung wenigstens eines leafing-Additivs überraschend gering ist.

Des weiteren können in der erfindungsgemäßen Zusammensetzung Zusätze wie Parfüme, Antioxidantien, Lichtschutzmittel oder Konservierungsstoffe enthalten sein. Hier werden übliche im Stand der Technik bekannte Substanzen eingesetzt.

### Beispiele

### Nagellackzusammensetzung

Erfindungsgemäßes Beispiel 1:

| **Nr.** | **Stoff** | **Konzentration in Gew.-%** |
|---|---|---|
| 1 | Metalure ® CA-41010 AE | 19 |
| 2 | Hostaphat CS 120 * | 1 |
| 3 | Methylethylketon | 20 |
| 4 | Methylisobutylketon | 20 |
| 5 | CAB 381.2 | 8 |
| 6 | Butylacetat 98/100 | 32 |

| | | |
|---|---|---|
| * Stearylphosphorsäureester | | |

### Herstellung:

Butylacetat wird vorgelegt und das CAB-Pulver unter Rühren portionsweise zugegeben.
Die Komponenten 2 - 4 werden nacheinander zu der Metalure ®-Dispersion gegeben und schonend eingearbeitet.

### Vergleichsbeispiel 2:

| **Nr.** | **Stoff** | **Konzentration in Gew.-%** |
|---|---|---|
| 1 | Metalure ® CA-41010 AE | 19 |
| 2 | Methylethylketon | 21 |
| 3 | Methylisobutylketon | 20 |
| 4 | CAB 381.2 | 8 |
| 5 | Butylacetat 98/100 | 32 |

### Herstellung: Analog zu Beispiel 1, jedoch ohne Zugabe von Hostaphat CS 120

### Vergleich und Beurteilung der Zusammensetzungen der Beispiele 1 und 2:

Visuelles Erscheinungsbild der Beispiele 1 und 2 nach Auftrag auf einen künstlichen Fingernagel:

| | |
|---|---|
| Erfindungsgemäßes Beispiel 1: | Hochglänzend silbrig, geschlossener Metallfilm ähnlich einem "flüssigen Metall". |
| | |
| Vergleichsbeispiel 2: | Glänzendes Silber, deutlich geringerer Glanz als Beispiel 1. |

## Patentansprüche

1. Kosmetische Zusammensetzung umfassend eine Flüssigphase und PVD-Aluminiumpigment, wobei die kosmetische Zusammensetzung PVD-Aluminiumpigment in einer Pigmentierungshöhe von 0,05 bis 5,0 Gew.-%, bezogen auf das Gesamtgewicht der kosmetischen Zusammensetzung, und wenigstens ein leafing-Additiv umfasst.

2. Kosmetische Zusammensetzung nach Anspruch 1,
**dadurch gekennzeichnet,**
**dass** das leafing-Additiv ein langkettiger Phosphorsäureester oder ein Gemisch aus mehreren langkettigen Phosphorsäureestern ist, wobei der bzw. die langkettigen Phosphorsäureester vorzugsweise eine Kohlenstoffkette mit einer Kettenlänge von 12 bis 24 Kohlenstoffatomen aufweist bzw. aufweisen.

3. Kosmetische Zusammensetzung nach Anspruch 2,
**dadurch gekennzeichnet,**
**dass** das leafing-Additiv ein langkettiger Phosphorsäureester oder ein Gemisch aus mehreren langkettigen Phosphorsäureestern ist, wobei der bzw. die langkettigen Phosphorsäureester vorzugsweise eine Kohlenstoffkette mit einer Kettenlänge von 14 bis 28 Kohlenstoffatomen aufweist bzw. aufweisen.

4. Kosmetische Zusammensetzung nach einem der vorhergehenden Ansprüche,
**dadurch gekennzeichnet,**
**daß** das leafing-Additiv ein Fettsäurephosphorsäureester oder ein Gemisch verschiedener Fettsäurephosphorsäureester ist.

5. Kosmetische Zusammensetzung nach einem der Ansprüche 1 bis 4,
**dadurch gekennzeichnet,**
**dass** die Pigmentierungshöhe des PVD-Aluminiumpigments 1 bis 2 Gew.-%, bevorzugt 1,3 bis 1,8 Gew.-% beträgt, jeweils bezogen auf das Gesamtgewicht der kosmetischen Zusammensetzung.

6. Kosmetische Zusammensetzung nach einem der Ansprüche 1 bis 5,
**dadurch gekennzeichnet,**
**dass** der Festkörpergehalt der kosmetischen Zusammensetzung zwischen 5 und 17 Gew.-% beträgt, bezogen auf das Gesamtgewicht der kosmetischen Zusammensetzung.

7. Kosmetische Zusammensetzung nach einem der Ansprüche 1 bis 6,
**dadurch gekennzeichnet,**
**dass** die kosmetische Zusammensetzung zusätzlich wenigstens eine filmbildende Komponente enthält und ein Nagellack ist.

8. Kosmetische Zusammensetzung nach Anspruch 7,
**dadurch gekennzeichnet,**
**dass** die filmbildende Komponente Nitrocellulose umfasst.

9. Kosmetische Zusammensetzung nach Anspruch 8,
**dadurch gekennzeichnet,**
**dass** die filmbildende Komponente Nitrocellulose mit einem Molekulargewicht von mehr als 56.000 g/mol, vorzugsweise von mehr als 112.000 g/mol, umfasst.

10. Kosmetische Zusammensetzung nach Anspruch 7,
**dadurch gekennzeichnet,**
**dass** die filmbildende Komponente Celluloseacetatbutyrat, das vorzugsweise ein Molekulargewicht von mehr als 83.000 g/mol aufweist, umfasst.

11. Kosmetische Zusammensetzung nach einem der vorstehenden Ansprüche,
**dadurch gekennzeichnet,**
**dass** die Flüssigphase ein organisches Lösemittel ist, das vorzugsweise aus der Gruppe, die aus Ketonen, Acetaten mit einem Molgewicht unter 120 g/mol und Gemischen davon besteht, ausgewählt wird.

12. Kosmetische Zusammensetzung nach einem der vorstehenden Ansprüche,
**dadurch gekennzeichnet,**
**dass** die Flüssigphase ein organisches Lösemittel ist, das vorzugsweise aus der Gruppe, die aus Methylisobutylketon, Methylethylketon, Ethylacetat, Butylacetat, Methylacetat, Aceton und Gemischen daraus besteht, ausgewählt wird.

13. Kosmetische Zusammensetzung nach Anspruch 12,
**dadurch gekennzeichnet,**
**dass** das organische Lösemittel Methylethylketon oder Ethylacetat ist.

14. Kosmetische Zusammensetzung nach einem der vorstehenden Ansprüche,
**dadurch gekennzeichnet,**
**dass** die kosmetische Zusammensetzung zusätzlich einen Weichmacher und/oder ein Dispergierungsmittel enthält.

15. Kosmetische Zusammensetzung nach Anspruch 14.
**dadurch gekennzeichnet,**
**dass** die kosmetische Zusammensetzung als Weichmacher wenigstens Dibutylphthalat, optional in Kombination mit Saccharoseacetatisobutyrat, enthält.

16. Kosmetische Zusammensetzung nach einem der vorstehenden Ansprüche,
**dadurch gekennzeichnet,**
**dass** die kosmetische Zusammensetzung als Dispergiermittel Bentonite, Stearylalkoniumhectorit oder Stearylalkoniumbentonit und/oder polymeren Harnstoff enthält.

17. Kosmetische Zusammensetzung nach einem der vorstehenden Ansprüche,
**dadurch gekennzeichnet,**
**dass** die kosmetische Zusammensetzung zusätzlich mindestens ein Farbmittel enthält.

18. Kosmetische Zusammensetzung nach Anspruch 17,
**dadurch gekennzeichnet,**
**dass** das Farbmittel ein Farbpigment und/oder Farbstoff ist.

19. Kosmetische Zusammensetzung nach einem der vorstehenden Ansprüche,
**dadurch gekennzeichnet,**
**dass** die kosmetische Zusammensetzung Zusatzstoffe, wie Thixotropiemittel, Antioxidantien, Emulgatoren, Vitamine, Duftstoffe, Lichtschutzmittel, Konservierungsstoffe, Füllstoffe und/oder Medikamente, enthält.

20. Kosmetische Zusammensetzung nach einem der vorstehenden Ansprüche,
**dadurch gekennzeichnet,**
**dass** die Pigmentierungshöhe der PVD-Aluminiumpigmente vorzugsweise 0,2 bis 2,0 Gew.-%, weiter bevorzugt 0,3 bis 1,0 Gew.-%, beträgt, jeweils bezogen auf das Gesamtgewicht der Zusammensetzung.

21. Künstlicher Fingernagel,
**dadurch gekennzeichnet,**
**dass** der künstliche Fingernagel mit einer kosmetischen Zusammensetzung nach einem der vorstehenden Ansprüche überzogen ist.

22. Verwendung von PVD-Aluminiumpigment mit leafing-Eigenschaften in einer kosmetischen Zusammensetzung, vorzugsweise einem Nagellack, wobei das PVD-Aluminiumpigment oberflächlich mit einem leafing-Additiv versehen ist.

## Claims

1. Cosmetic composition comprising a liquid phase and an aluminium pigment produced by PVD, which cosmetic composition contains PVD aluminium pigment in a pigment quantity of 0.05 to 5.0 % by weight, based on the total weight of the cosmetic composition, and at least one leafing additive.

2. Cosmetic composition as claimed in claim 1,
**characterised in that**
the leafing additive is a long-chained phosphoric acid ester or a mixture of several long-chained phosphoric acid esters, and the long-chained phosphoric acid ester or esters preferably has/have a carbon chain with a chain length of 12 to 24 carbon atoms.

3. Cosmetic composition as claimed in claim 2,
**characterised in that**
the leafing additive is a long-chained phosphoric acid ester or a mixture of several long-chained phosphoric acid esters, and the long-chained phosphoric acid ester or esters preferably has/have a carbon chain with a chain length of 14 to 28 carbon atoms.

4. Cosmetic composition as claimed in one of the preceding claims,
**characterised in that**
the leafing additive is a fatty acid phosphoric acid ester or a mixture of different fatty acid phosphoric acid esters.

5. Cosmetic composition as claimed in one of claims 1 to 4,
**characterised in that**
the pigment quantity of the PVD aluminium pigment is 1 to 2 % by weight, preferably 1.3 to 1.8 % by weight, based respectively on the total weight of the cosmetic composition.

6. Cosmetic composition as claimed in one of claims 1 to 5,
**characterised in that**
the solids content of the cosmetic composition is between 5 and 17 % by weight, based on the total weight of the cosmetic composition.

7. Cosmetic composition as claimed in one of claims 1 to 6,
**characterised in that**
the cosmetic composition additionally contains at least one film-forming component and is a nail varnish.

8. Cosmetic composition as claimed in claim 7,
**characterised in that**
the film-forming component is nitrocellulose.

9. Cosmetic composition as claimed in claim 8,
**characterised in that**
the film-forming component is nitrocellulose with a molecular weight of more than 56,000 g/mol, preferably more than 112,000 g/mol.

10. Cosmetic composition as claimed in claim 7,
**characterised in that**
the film-forming component is cellulose acetate butyrate, which preferably has a molecular weight of more than 83,000 g/mol.

11. Cosmetic composition as claimed in one of the preceding claims,
**characterised in that**
the liquid phase is an organic solvent which is preferably selected from the group comprising ketones, acetates with a molecular weight of less than 120 g/mol and mixtures thereof.

12. Cosmetic composition as claimed in one of the preceding claims,
**characterised in that**
the liquid phase is an organic solvent which is preferably selected from the group comprising methylisobutyl ketone, methylethyl ketone, ethyl acetate, butyl acetate, methyl acetate, acetone and mixtures thereof.

13. Cosmetic composition as claimed in claim 12,
**characterised in that**
the organic solvent is methylethyl ketone or ethyl acetate.

14. Cosmetic composition as claimed in one of the preceding claims,
**characterised in that**
the cosmetic composition additionally contains a plasticizer and/or a dispersing agent.

15. Cosmetic composition as claimed in claim 14,
**characterised in that**
the cosmetic composition at least dibutyl phthalate, optionally in combination with saccharose acetate isobutyrate, as plasticizers.

16. Cosmetic composition as claimed in one of the preceding claims,
**characterised in that**
the cosmetic composition contains bentonite, stearyl alkonium hectorite or stearyl alkonium bentonite and/or polymeric resin as dispersing agents.

17. Cosmetic composition as claimed in one of the preceding claims,
**characterised in that**
the cosmetic composition additionally contains at least one colouring agent.

18. Cosmetic composition as claimed in claim 17,
**characterised in that**
the colouring agent is a coloured pigment and/or dye.

19. Cosmetic composition as claimed in one of the preceding claims,
**characterised in that**
the cosmetic composition contains additives such as thixotropic agents, antioxidants, emulsifiers, vitamins, perfumes, light-stabilising agents, preservatives, fillers and/or medicaments.

20. Cosmetic composition as claimed in one of the preceding claims,
**characterised in that**
the pigment quantity of the PVD aluminium pigments is preferably 0.2 to 2.0 % by weight, more preferably 0.3 to 1.0 % by weight, based respectively on the total weight of the composition.

21. Artificial finger nail,
**characterised in that**
the artificial finger nail is coated with a cosmetic composition as claimed in one of the preceding claims.

22. Use of PVD aluminium pigment with leafing properties in a cosmetic composition, preferably a nail varnish, and the PVD aluminium pigment is provided with a leafing additive on its surface.

## Revendications

1. Composition cosmétique comprenant une phase liquide et un pigment d'aluminium PVD, ladite composition cosmétique comprenant un pigment d'aluminium PVD à un degré de pigmentation compris entre 0,05 et 5,0% en poids, ramené au poids total de la composition cosmétique, et au moins un additif pelliculant.

2. Composition cosmétique selon la revendication 1, **caractérisée en ce que** l'additif pelliculant est un ester à chaîne longue de l'acide phosphorique ou un mélange de plusieurs esters à chaîne longue de l'acide phosphorique, le ou les esters à chaîne longue de l'acide phosphorique présentant de préférence une chaîne carbonée dont la longueur de chaîne va de 12 à 24 atomes de carbone.

3. Composition cosmétique selon la revendication 2, **caractérisée en ce que** l'additif pelliculant est un ester à chaîne longue de l'acide phosphorique ou un mélange de plusieurs esters à chaîne longue de l'acide phosphorique, le ou les esters à chaîne longue de l'acide phosphorique présentant de préférence une chaîne carbonée dont la longueur de chaîne va de 14 à 28 atomes de carbone.

4. Composition cosmétique selon l'une des revendications précédentes, **caractérisée en ce que** l'additif pelliculant est un ester phosphate d'acide gras ou un mélange de différents esters phosphates d'acides gras.

5. Composition cosmétique selon l'une des revendications 1 à 4, **caractérisée en ce que** le degré de pigmentation du pigment d'aluminium PVD est compris entre 1 et 2% en poids, de préférence entre 1,3 et 1,8% en poids, ramené à chaque fois au poids total de la composition cosmétique.

6. Composition cosmétique selon l'une des revendications 1 à 5, **caractérisée en ce que** la teneur en solides de la composition cosmétique est comprise entre 5 et 17% en poids, ramenée au poids total de la composition cosmétique.

7. Composition cosmétique selon l'une des revendications 1 à 6, **caractérisée en ce que** la composition cosmétique contient en plus au moins un composant filmogène et est un vernis à ongles.

8. Composition cosmétique selon la revendication 7, **caractérisée en ce que** le composant filmogène comprend de la nitrocellulose.

9. Composition cosmétique selon la revendication 8, **caractérisée en ce que** le composant filmogène comprend de la nitrocellulose ayant une masse moléculaire supérieure à 56 000 g/mol, de préférence supérieure à 112 000 g/mol.

10. Composition cosmétique selon la revendication 7, **caractérisée en ce que** le composant filmogène comprend de l'acétate butyrate de cellulose qui présente de préférence une masse moléculaire supérieure à 83 000 g/mol.

11. Composition cosmétique selon l'une des revendications précédentes, **caractérisée en ce que** la phase liquide est un solvant organique qui est de préférence choisi dans le groupe comprenant des cétones, des acétates de masse molaire inférieure à 120 g/mol, et des mélanges de ceux-ci.

12. Composition cosmétique selon l'une des revendications précédentes, **caractérisée en ce que** la phase liquide est un solvant organique qui est de préférence choisi dans le groupe comprenant la méthylisobutylcétone, la méthyléthylcétone, l'acétate d'éthyle, l'acétate de butyle, l'acétate de méthyle, l'acétone et des mélanges de ceux-ci.

13. Composition cosmétique selon la revendication 12, **caractérisée en ce que** le solvant organique est la méthyléthylcétone ou l'acétate d'éthyle.

14. Composition cosmétique selon l'une des revendications précédentes, **caractérisée en ce que** la composition cosmétique contient en plus un plastifiant et/ou un agent dispersant.

15. Composition cosmétique selon la revendication 14, **caractérisée en ce que** la composition cosmétique contient comme plastifiant au moins du phtalate de dibutyle, le cas échéant en combinaison avec de l'acétate isobutyrate de saccharose.

16. Composition cosmétique selon l'une des revendications précédentes, **caractérisée en ce que** la composition cosmétique contient comme agent dispersant des bentonites, de la stéarylalkonium hectorite ou de la stéarylalkonium bentonite et/ou de l'urée polymère.

17. Composition cosmétique selon l'une des revendications précédentes, **caractérisée en ce que** la composition cosmétique contient en plus au moins une matière colorante.

18. Composition cosmétique selon la revendication 17, **caractérisée en ce que** la matière colorante est un pigment coloré et/ou un colorant.

19. Composition cosmétique selon l'une des revendications précédentes, **caractérisée en ce que** la composition cosmétique contient des substances ajoutées telles que des agents thixotropes, des antioxydants, des émulsifiants, des vitamines, des parfums, des agents photoprotecteurs, des conservateurs, des matières de charge et/ou des médicaments.

20. Composition cosmétique selon l'une des revendications précédentes, **caractérisée en ce que** le degré de pigmentation des pigments d'aluminium PVD est de préférence compris entre 0,2 et 2,0% en poids, de façon plus préférentielle entre 0,3 et 1,0% en poids, ramené à chaque fois au poids total de la composition.

21. Ongle artificiel, **caractérisé en ce que** ledit ongle artificiel est revêtu avec une composition cosmétique selon l'une des revendications précédentes.

22. Utilisation d'un pigment d'aluminium PVD ayant des propriétés pelliculantes dans une composition cosmétique, de préférence un vernis à ongles, le pigment d'aluminium PVD étant muni en surface d'un additif pelliculant.
